# EUROPEAN PATENT SPECIFICATION

(11) **EP 0 421 558 B1**
(45) Date of publication and mention of the grant of the patent: **26.04.1995**
(21) Application number: 90202829.9
(22) Date of filing: 23.07.1984
(51) Int. Cl.: A61M 1/00, A61F 2/00, H02K 37/00

(54) **A surgically implantable device**
Chirurgisch implantierbare Vorrichtung
Dispositif chirurgical implantable

(30) Priority: 21.07.1983 US 516137; 08.12.1983 US 559864; 08.12.1983 US 559865
(43) Date of publication of application: 10.04.1991
(62) Divisional of application: 84304995.8
(73) Proprietor: Hakim, Salomon, Bogota (CO); Hakim, Carlos A., Fort Lauderdale Florida 33308 (US)
(72) Inventor: Hakim, Salomon, Bogota (CO); Hakim, Carlos A., Fort Lauderdale Florida 33308 (US)
(74) Representative: Deans, Michael John Percy

(56) References cited:
- FR-A- 2 354 103
- US-A- 3 886 948
- US-A- 4 387 715

## Description

This invention relates to surgically-implanted devices, namely shunt valves for venting cerebrospinal fluid ("CSF") in the treatment of hydrocephalus and similar conditions of impaired circulation and absorption of body fluids.

Cerebrospinal fluid shunt valves have been in use for over twenty years. Broadly speaking, they function by venting excess cerebrospinal fluid from the brain into the venous system or other receptive cavities (e.g., peritoneal, pleural). Many such valves, including the earliest designs, operate by controlling the amount of fluid flow. The neurosurgeon makes an estimate of the amount of flow required to relieve the hydrocephalus and selects a valve of that flow capacity. The selection is made difficult by the wide variation in normal flow rates.

About twenty years ago, one of the present applicants, namely Salomon Hakim, developed an altogether different valve, one that controlled intraventricular pressure rather than flow. That valve, which is today known as the Cordis-Hakim shunt valve, and which is described in U.S. Patent No. 3,288,142 has been enormously successful and remains, even today, one of the most popular shunt valves in use. It has a spherical sapphire ball biased against a conical valve seat by a stainless steel spring. The pressure of cerebrospinal fluid pushes against the sapphire ball and spring in a direction tending to raise the ball from the seat.
When the pressure difference across the valve (e.g., the pressure difference between the cerebral ventricle and the drainage site) exceeds a so-called popping pressure, the ball rises from the seat to vent cerebrospinal fluid. As the flow rate through the valve increases, the ball moves further away from the seat to provide a larger valve orifice, one that is always large enough that the pressure drop across the orifice never rises much above the popping pressure. Accordingly, the differential pressure across the valve remains nearly constant for any flow rate encountered within the cerebrospinal fluid system.

As successful as the Cordis-Hakim valve has been, it has one important limitation. It can only provide a fixed popping pressure. In treating hydrocephalus, it is often desirable to vary the popping pressure in accordance with ventricle size and treatment objective. For example, initial treatment may require a lower than normal pressure to initiate shrinkage of the ventricles, but as the ventricles decrease in size, the popping pressure should be increased gradually so that when the ventricles return to normal size the intraventricular pressure is at its normal value and the intracranial force systems are in balance (i.e., the popping pressure is set at a level that will stabilize the ventricles at a desired size). Generally speaking, the popping pressure should be varied inversely with the ventricle size. It is undesirable to leave a low pressure valve in a patient after the ventricles are again normal size, because the ventricles can further collapse, leading to a condition known as "slit" ventricles. A fuller discussion of these matters can be found in Hakim et al., "A Critical Analysis of Valve Shunts Used in the Treatment of Hydrocephalus", Developmental Medicine and Child Neurology, Vol. 15, No. 2, April 1973, pp. 230-255.

A further reason for providing adjustablility in popping pressure is to correct for the wide variation in nominal popping pressure typical in manufactured valves. With an adjustable valve, the popping pressure can be more accurately set at the factory, and can be checked, and corrected if necessary, in the operating room prior to implantation. Moreover it is unnecessary to manufacture and stock valves with differing nominal pressures, as one valve can typically provide all desired pressures according to the needs at any given moment of the treatment.

Efforts have been made at developing an adjustable valve. An example is a valve disclosed in our earlier-filed copending United States application Serial No. 493,748, in which an adjustment screw is turned either by a screw driver applied through the skin to the valve or by rotation of a magnet along an axis aligned with the axis of the screw.

Our US Patent 4 387 715 also provides for mechanical adjustment of pressure by means of a screw turned by an external screwdriver. The said US-A-4 387 715 discloses a shunt valve according to the preamble of Claims 1 and 30.

Purely magnetic adjustment of implanted hydrocephalus shunt valves has been previously proposed in FR-A-2 354 103, in which an externally applied passive magnetic field is used to attract a magnetic disc of the implanted device to cause it to move axially, thereby allowing a plate serving as a shunt valve member to lift from its seat, and by lever action to open a further valve to a drain.

Implantable magnetically-driven devices have been proposed. Levy et al. U.S. Patent No. 4,360,007 discloses an implantable actuator with a ratchet wheel, pawl, and permanent magnet; application of an external magnetic field rotates the implanted magnet and pawl to advance the ratchet wheel.

Stepping motors have been known per se for many years. The simplest stepping motor consists of a permanent magnet rotor surrounded by a stator made up of four electromagnets. By selectively energizing the electromagnets, it is possible to turn the rotor in angular "steps" of 90°. Often the rotor has a plurality of permanent magnets, so as to reduce the angular step size. Some stepping motors replace the permanent magnets on the rotor with regions of different magnetic reluctance; in these, known as variable reluctance motors, the rotor turns to the position of minimum reluctance. Still others, known as hybrids, combine reluctance differences with permanent magnets.

Stepping motors have been used in some medical applications. For example, they have been used in medical infusion pumps for delivering precise volumetric dosages of drugs at prescribed time intervals. In all these applications the stepping motor was located outside the patient's body, either in a portable device carried by the patient or in a bedside unit.

Other types of medical devices (e.g., pacemakers) have been implanted in the body. These have typically relied for a power source on batteries implanted along with the device.

In accordance with a first aspect of the present invention, we provide for venting cerebrospinal fluid in the treatment of hydrocephalus or for shunting other body fluids, a surgically-implantable shunt valve comprising: a housing constructed of a surgically-implantable material; an inlet and outlet chamber within said housing, said housing including inlet and outlet ports communicating with said inlet and outlet chambers respectively for connecting said inlet and outlet chambers to external catheters or other fluid conduits; an aperture communicating between said chambers, said aperture having a circular periphery forming a circular valve seat; a valve member of diameter larger than said circular valve seat such that it fits into said circular valve seat so as to seal it; and spring means for biasing said valve member against the circular valve seat, said spring means being adapted to keep said aperture closed until fluid pressure in said inlet chamber exceeds a preselected popping pressure and adapted to open said aperture when said popping pressure is exceeded so as to vent fluid through said aperture into said outlet chamber; said valve being characterised in further comprising incremental magnetic adjustment means adapted to increase or decrease the amount of said bias in finite increments in response to pulses of magnetic field applied from outside a body in which the valve is implanted, so as thereby to increase or decrease said popping pressure in finite increments, said incremental magnetic adjusting means comprising a primary member adapted to co-operate with said spring means so as to increase or decrease said bias when said primary member is moved, said primary member not itself being susceptible to movement by said external magnetic field, and a secondary member susceptible to said applied magnetic field and mounted and positioned with respect to said primary member so as to be capable of movement relative to said primary member and so as to cause no more than an incremental movement of said primary member for each pulse of said magnetic field.

According to a second and alternative aspect of the invention, we provide for venting cerebrospinal fluid in the treatment of hydrocephalus or for shunting other body fluids, a surgically-implantable shunt valve comprising: a housing constructed of a surgically-implantable material; an inlet and outlet chamber within said housing, said housing including inlet and outlet ports communicating with said inlet and outlet chambers respectively for connecting said inlet and outlet chambers to external catheters or other fluid conduits; an aperture communicating between said chambers, said aperture having a circular periphery forming a circular valve seat; a valve member of diameter larger than said circular valve seat such that it fits into said circular valve seat so as to seal it; and spring means for biasing said valve member against the circular valve seat, said spring means being adapted to keep said aperture closed until fluid pressure in said inlet chamber exceeds a preselected popping pressure and adapted to open said aperture when said popping pressure is exceeded so as to vent fluid through said aperture into said outlet chamber; said valve being characterised in further comprising incremental magnetic adjustment means adapted to increase or decrease the amount of said bias in finite increments in response to pulses of an applied magnetic field so as thereby to increase or decrease said popping pressure in finite increments, said incremental magnetic adjusting means comprising an element adapted to rotate in response to an external magnetic field, and a cam in the shape of a circular staircase, said spring means including an arm supported on said staircase.

The incremental magnetic adjustment means may comprise a stepping motor which is isolated physically from electrical power sources and adapted to be powered by the influence of a magnetic field applied from outside the apparatus, said stepping motor comprising a rotor and one or more stator elements, said stator elements being composed of magnetically soft and permeable material shaped and positioned with respect to said rotor so that when magnetized under the influence of said external field the said stator elements strengthen and orient the magnetic field in their vicinities so as to cause movement of said rotor.

The stepping motor may be operated while totally isolated physically from any source of electrical power (i.e.,without batteries and without wire connections to an external power source). The usual electromagnets used for the stator are replaced by pieces of magnetically soft and permeable material (e.g., pure iron or special alloys such as vacoperm). The externally applied magnetic field is used to magnetize the stator elements so that the localized magnetic field in the vicinity of the stator elements causes rotation of the rotor. The external magnetic field need not be applied with great precision, as the stator elements strengthen and orient the effect of the magnetic field in their vicinities.

In preferred embodiments, the rotor has a plurality of permanently magnetic poles; there are a plurality of stator elements spaced around the rotor or one stator element with a plurality of lobes so spaced; the external magnetic field is applied using apparatus having a plurality of electromagnets, which are equal in number to the stator elements (or lobes of one element) and positioned similarly; and the stator elements are composed either of soft and permeable material or of such material wrapped by an electrical coil (in which is induced an electrical current, which in turn helps magnetize the soft, permeable stator material).

It will be appreciated that as a result, a mechanical movement (e.g., rotary or linear) is induced within the implanted valve to alter the pressure setting thereof without any physical connection (e.g., electrical wires) with the valve. It makes possible implanting the valve so that its popping pressure can be accurately adjusted without the need for any wires, tubes, or other physical elements penetrating through the skin, or the use of implanted batteries.

Thus, it will be see that in our surgically-implantable shunt valve the popping pressure is adjusted in finite increments by application of an external magnetic field. In preferred embodiments, the magnetic field causes rotation of a member, which, in turn, moves a portion of the spring biasing the ball against the valve seat; each pulse or cycle of the magnetic field causes one incremental change in the popping pressure (e.g., ten or more cycles are required to move from minimum to maximum pressure); a toothed gear is moved by a rotating escapement element, which is driven by the magnetic field; the gear is connected to a screw or cam that, in turn, changes the bias setting of the spring; in some embodiments, a ferromagnetic element or permanent magnet is mounted on an arm extending radially from said escapement element, a spring is provided to return the arm and escapement to a neutral position, and application of a single external magnetic pulse causes the arm and escapement to move away from and then back to the neutral position sufficiently to cause an incremental rotation of the gear; in other embodiments, two escapement elements are provided, each attached to a magnet and pivoted about an axis passing between the poles of the magnet; the axes of rotation of the two escapement elements are separated around the gear by a whole number of teeth plus one-half tooth; the escapement elements and magnets are either about 180° apart or about 90° apart; the external adjusting electromagnets are aligned with the magnets inside the valve; the magnetic field generated by the external electromagnets is strongest at the magnet and escapement closest to the energized magnet and thus the closest magnet is dominant and controls movement of the gear; the escapement elements have teeth on either end that mesh with the teeth of the gear; the teeth are either of the same general shape as the gear teeth (e.g., blunt) or are long, thin elements adapted to pierce through debris that collects between the gear teeth; a wheel replaces the gear and the wheel has a plurality of permanent magnets arranged around its axis of rotation, adjacent magnets having opposite polarity; the external adjusting magnets are positioned so that one is aligned with one of the permanent magnets when the other is midway between two of the permanent magnets, so that alternate energizing of the external magnets will advance the wheel one-half the angular spacing of the magnets for each energization; in some embodiments, a movable external magnet is swept by the permanent magnets (e.g., by rotation about an axis parallel to the wheel axis and radially outside the permanent magnets) to cause the wheel to rotate; and a stop is provided for the gear at a position corresponding to a known popping pressure, to permit the gear first to be adjusted against the stop and then adjusted in increments away therefrom.

In an alternative arrangement, a three-arm spring is used in conjunction with magnetic adjustment. The spring has two flanking arms retained at their ends and a central arm that biases the ball of the valve at one end and is moved in response to the magnetic adjustment at the other end.

In another arrangement, a yoke retains such a three-arm spring. In preferred embodiments, the yoke extends alongside the inner edges of the flanking arms ( to provide sideward retention for the spring) and over the top surface of those arms (to provide upward retention); the yoke is U-shaped to pass under the central arm and upwardly between the central and flanking arms; and notches are provided in flanking arms to receive portions of the yoke (to provide longitudinal retention).

The invention in its second aspect has the advantages of immunity to the magnetic field generated by NMR (nuclear magnetic resonance) diagnostic devices; such devices generate a steady magnet field, which cannot cause more than about one incremental change of popping pressure. It is also resistant to other incidental magnetic fields because repetitive, strong magnetic pulses are required to cause a substantial adjustment to popping pressure. The invention provides a simple, reliable and accurate technique for noninvasive adjustment of popping pressure.

The invention is hereinafter more particularly described by way of example only with reference to the accompanying drawings.

Fig. 1 is a perspective, somewhat diagrammatic, view of a first preferred embodiment of the invention.

Fig. 2 is a cross-sectional view taken at 2-2 of Fig. 1, showing the internal construction of said first embodiment.

Figs. 3a and 3b are views taken along 3-3 in Fig. 2 showing two positions of the magnetic adjustment arm of said first embodiment.

Fig. 4 is a view taken along 4-4 in Fig. 2 of the ball biasing spring of said first embodiment.

Fig. 5 is a cross-sectional, somewhat diagrammatic, view taken along 5-5 of Fig. 2 showing the valve as it would appear installed on the skull and also showing the valve adjustment element positioned over the valve.

Fig. 6 is a plan view, partially cutaway, of a second preferred embodiment of the invention, showing the interior structure of the valve.

Fig. 7 is a cross-sectional view taken along 7-7 of Fig. 6.

Fig. 8 is a cross-sectional view taken along 8-8 of Figs. 6 and 7.

Fig. 8a is a plan view of the three-arm spring of the valve.

Fig. 9 is a partial plan view of said second embodiment showing only the magnetic escapement elements and gear.

Figs. 10a to 10d are diagrammatic plan views of the escapement mechanism of said second embodiment, showing that mechanism in four different stages in one full cycle thereof.

Fig. 11 is a partial plan view of a preferred variation of the gear and escapement portion of said second embodiment.

Fig. 12 is a partial sectional view of a preferred variation of the cam and gear of said second embodiment.

Fig. 13 is a diagrammatic view of the gear and escapement element of another variation of said second embodiment.

Figs. 14, 15, and 16 are diagrammatic views of the magnet wheels and external adjusting magnets of three other embodiments.

Fig. 17 is a perspective, somewhat diagrammatic, view of the most preferred embodiment of the invention.

Fig. 18 is a cross-sectional view taken at 18-18 of Fig. 17, showing the internal construction of said embodiment.

Fig. 19 is a cross-sectional view taken at 19-19 in Fig. 18.

Fig. 20 is an elevation view taken at 20-20 in Fig. 18.

Fig. 21 is a plan view at 21-21 in Fig. 18, showing the cam of said embodiment.

Fig. 22 is a cross-sectional view of said cam taken at 22-22 in Fig. 21.

Fig. 23 is a diagrammatic view of the steps of said cam.

Fig. 24 is a plan view of the internal support plate of said embodiment.

Fig. 24A is a cross-sectional view taken at 24A-24A in Fig. 24.

Fig. 25 is a plan view of the permanent-magnet disk of said embodiment, showing the ten pairs of poles on said disk.

Fig. 26 is a cross-sectional view of said disk taken at 26-26 in Fig. 25.

Fig. 27 is a diagrammatic view of said embodiment in which the positions of the external adjusting electromagnets are shown (much smaller than actual size).

Fig. 28 is a diagrammatic view of said embodiment implanted beneath the scalp and covered by an external adjustment element.

Fig. 29 is a diagrammatic view similar to Fig. 27 except that the rotor and cam have been removed to show the magnetic polarization of the four stator elements.

Figs. 30 and 31 show the magnetic polarization of an alternative embodiment, where a one-piece stator element is used.

Fig. 32 is a partial plan view, somewhat diagrammatic, of an alternative embodiment wherein the stator elements each include an electrical coil.

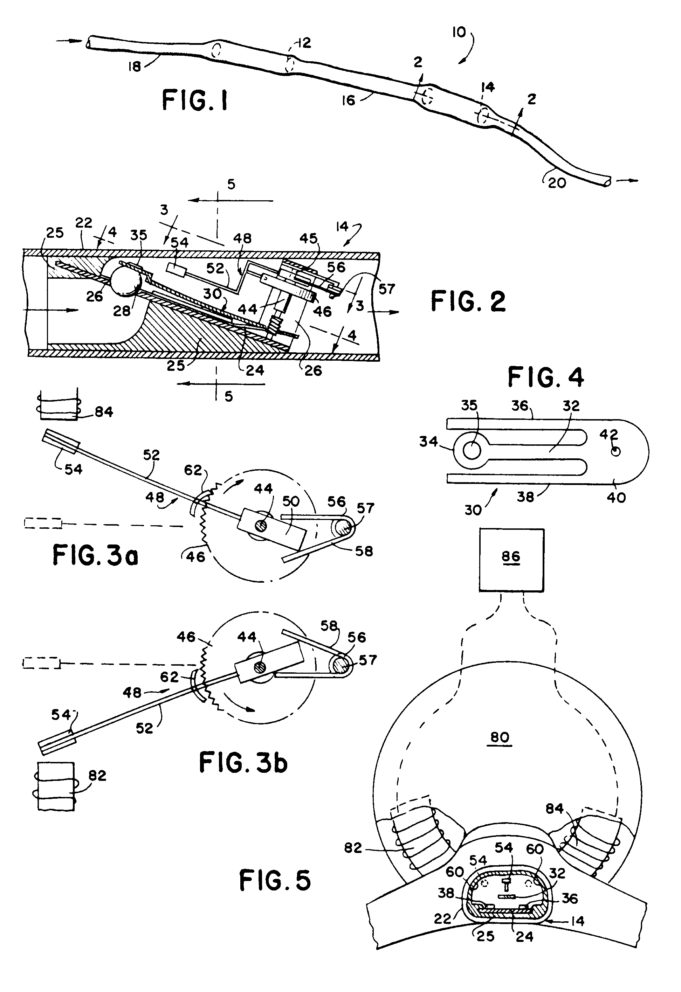

There is shown in Fig. 1 a shunt valve assembly 10 with two shunt valves 12, 14 separated by a pumping chamber 16. Cerebroventricular catheter 18 is connected to the inlet of the valve assembly, and drainage catheter 20, to the outlet. This assembly can be surgically implanted following well-known procedures.

A cross section through the downstream shunt valve 14 is shown in Fig. 2 (the upstream valve 12 is preferably the same except that the adjustment mechanism is absent). Valve body 22 (a surgically implantable material such as polyethersulfone or stainless steel) has within its interior an inclined plate 24 (stainless steel) held in place by molded portions 25 (plastic molded in place using forms inserted within the valve and subsequently removed). Formed in plate 24 is a circular aperture 26, the periphery of which forms a valve seat for spherical ball 28 (highly-polished sapphire). The periphery has a coined, beveled edge.

Biasing the ball against the valve seat is spring 30 (single piece of stainless steel), which is shown in plan view in Fig. 4. The spring has central arm 32 with enlarged end 34 with dimple 35 overlying and capturing ball 28. The central arm is joined to two flanking arms 36, 38 at a rear portion 40, in which there is provided hole 42 for receiving the threaded end of shaft 44.

Shaft 44 is supported at one end in a hole formed in plate 24, and at the other end in a hole formed in upper, integral extension 45 of plate 24. Fixed to the shaft at the end opposite the spring is toothed gear 46. Freely mounted to the shaft above the gear is magnetic adjustment arm 48, consisting of base 50 (stainless steel) mounted to the shaft, extension arm 52 (which extends from the base and has a step shape to pass around gear 46), and ferro-magnetic element 54 (or permanent magnet). Torsion spring 56 (stainless steel) with portions 58 flanking each side of base 50 serves to urge the arm 48 back to its equilibrium position (shown in dashed lines in Fig. 3). The torsion spring is mounted around stub shaft 57, which is secured to plate extension 45.

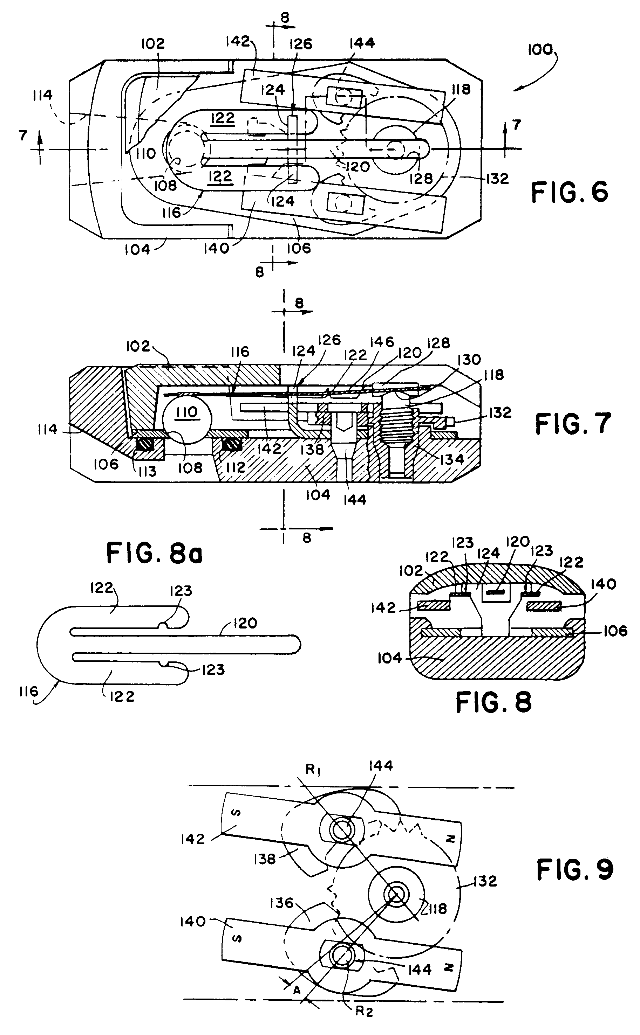

Turning to Figs. 6-10, there is shown a second preferred embodiment of the invention. Assembly 100 shown in the figures would replace downstream valve 14 shown in Figs. 1-5. A tubular plastic covering would be fitted tightly around the exterior of the assembly. A similar assembly, but absent the adjustment means, would replace upstream valve 12.

Upper and lower housings 102, 104 (molded of plastic, a product of Bayer AG) are ultrasonically welded together so as to clamp therebetween plate 106 (stainless steel). Circular aperture 108 in plate 106 provides a valve seat for spherical ball 110 (highly-polished sapphire). O-ring 112 installed in groove 113 in lower housing 104 provides a seal between the lower housing and the plate in the vicinity of the valve seat, to ensure that all flow through the valve is through the orifice formed between ball 110 and the valve seat 108. Cavity 114 in lower housing 104 provides the flow path for fluid upstream of the valve seat.

Ball 110 is biased against the valve seat by spring 116, which is shown in plan view in Fig. 6, and which has a central arm 120, extending from above the ball to an adjustment screw 118 at the other end of the assembly, and two flanking arms 122, the ends of which are held beneath the arms 124 of yoke 126, which is U-shaped at its upper end so as to pass around central arm 120, and which is an integral extension of plate 106 (all of which is shown most clearly in Fig. 8). Notches 123 cut in the ends of flanking arms 122 (Fig. 8a) receive the yoke arms 124, and secure the spring against longitudinal movement. The spring is secured against sideward movement by contact with the vertical outside surfaces of yoke 126.

Spring arm 120 is received in slot 128 in the head of screw 118, and rests on a wedge-shaped protrusion 130 within the slot. In that way, the screw is prevented from turning, and the force applied to the spring by the screw is transferred in a vertical direction at the center of the spring arm. The height of screw 118, and thus the amount of force applied to the spring, is adjusted by turning gear 132, which is threaded to screw 118. Gear 132 has integral shaft 134 extending into lower housing 104. Screw 118 is threaded into the interior of integral shaft 134.

Gear 132, which has 14 teeth, is rotated by movement of escapement elements 136, 138 (best shown in Fig. 9). The escapement elements, which are mirror images of one another, are keyed to permanent magnets 140, 142, and each escapement-magnet pair is supported for free rotation on a shaft 144 supported in lower housing 104. The escapement elements are prevented from falling out of the lower housing by protrusions 146 (Fig. 7), which extend down over the heads of the shafts from upper housing 102. The centers of rotation of the escapement elements are positioned along radial lines R₁ and R₂, which are separated by an angle corresponding to a whole number of teeth plus one half of a tooth of gear 132 (the number of integral teeth can be varied, but it is preferred that there remain an additional one-half tooth separation). Both magnets 140, 142 are oriented so that their north-south axes have the same polarity and are parallel when the escapement teeth are positioned relative to the gear teeth as shown in Fig. 9.

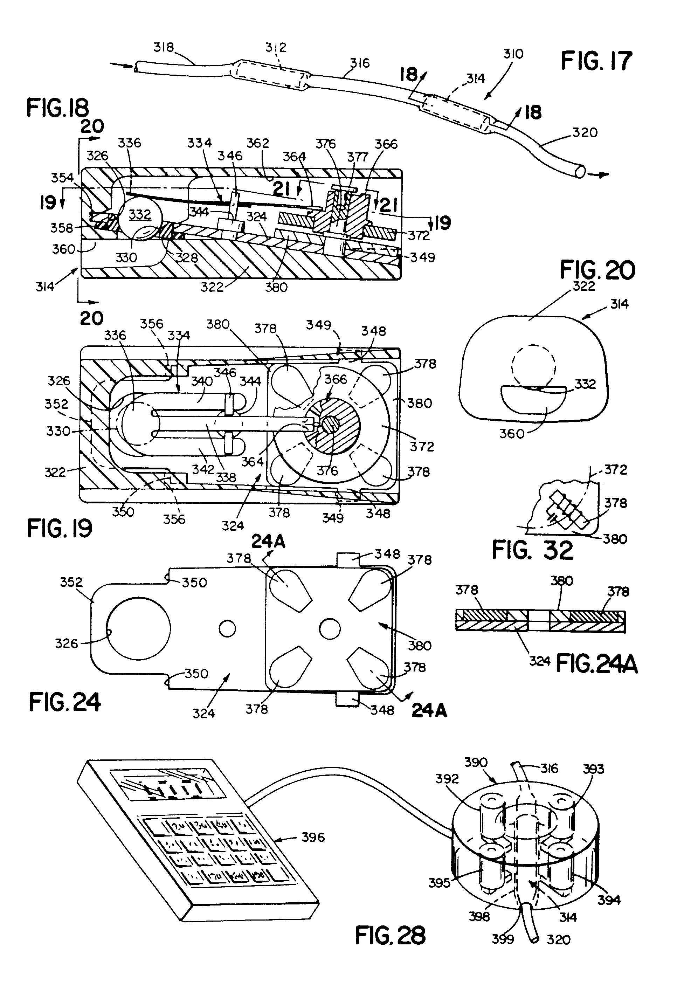

Turning to Figs. 17-32, there is shown another embodiment of the invention.

There is shown in Fig. 17 a shunt valve assembly 310 with two shunt valves 312, 314 separated by a pumping chamber 316. Cerebroventricular catheter 318 is connected to the inlet of the valve assembly, and drainage catheter 320, to the outlet. This assembly can be surgically implanted following well-known procedures.

A cross section through the downstream shunt valve 314 is shown in Fig. 18. The upstream valve 312 is preferably the same except that the adjustment mechanism is absent. (The tubular plastic covering shown tightly fitted around the valves in Fig. 17 is not shown in the remaining figures.) Valve body 322 (injection molded from a surgically-implantable material such as polyethersulfone) has within its interior an inclined plate 324 made from a nonmagnetic material, such as titanium or stainless steel. The plate 324 has circular aperture 326 in which is press fit a sapphire ring 328, with frustoconical surface 330 forming a valve seat for spherical ball 332 (highly-polished ruby).

Biasing the ball against the valve seat is spring 334 (single piece of stainless steel or another suitable material), shown in plan view in Fig. 19. The spring provides a low K factor to produce little change in working pressure with changes in flow (i.e., a flat flow-pressure curve). The spring has base 336 overlying ball 332, central arm 338 extending from the base to an adjustment mechanism, and two flanking arms 340, 342 extending from the base to a yoke 344. The yoke is press fit into a hole in plate 324 and tabs 346 extend over the tops of the flanking arms. The yoke is relieved in the center to provide room for the central arm to pass through. Notches (not shown) cut in the ends of flanking arms 340, 342 receive portions of the yoke, and secure the spring against longitudinal movement. The spring is secured against sideward movement by contact of the flanking arms with the vertical outside surfaces of the yoke.

Plate 324 is held tightly in place within valve body 322. The tight fit is achieved by sliding the plate into the valve body (in a direction from right to left in Fig. 18). Grooves 354, 356 at the upstream end of the valve body receive portions 350, 352 (Fig. 24) of the plate, and grooves 349 at the downstream end receive tabs 348 on the plate. The grooves extend generally horizontally rather than in the inclined direction followed by the plate, and thus the tabs 348 and portions 350, 352 tend to become tightly wedged into the grooves.

Grooves 354, 356 at the ball end of the valve body also serve to press plate 324 downwardly so as to squeeze it tightly against O-ring 358 (silicone rubber), which provides an internal seal to ensure that all flow through the valve is through the orifice formed between the ball 332 and the valve seat 330. Flow through the valve is from inlet cavity 360, past ball 332, and into outlet cavity 362.

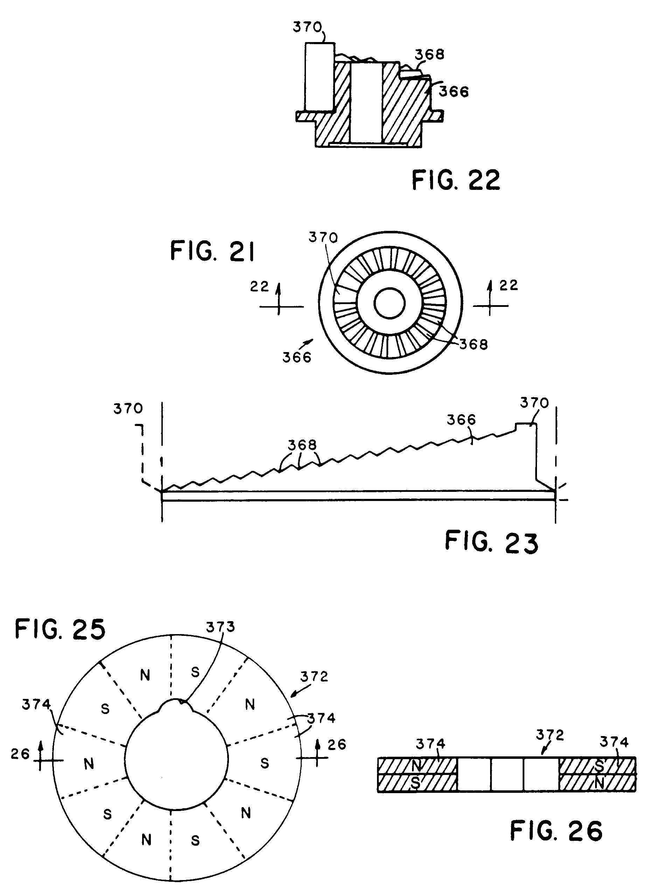

The preload of spring 334 against ball 332 is adjusted by using cam 366 (Delrin) to vary the vertical position (through a 0.75 mm range) of free end 364 of central arm 338. The spring preload establishes the pressure of the valve. The cam (best shown in Figs. 21-23) has a circular staircase of eighteen steps, each being grooved so as to have a V-shape cross section. Free end 364 of arm 338 has a similar V-shape chosen to mate with the V-shape of steps 368. At each end of the staircase a barrier is provided by element 370. This confines rotation of the cam to slightly less than one revolution. The V-shape of steps 368 act as detents to keep the cam in precisely one of eighteen possible angular positions. That means that the vertical position of free end 364 of arm 338 is always at precisely one of eighteen different values and, in turn, that the working pressure of the valve is always at one of eighteen possible levels.

Cam 366 is press fit into the central hole in rotor 372 (4 mm diameter), with a protrusion on the cam fitting into recess 373 in the rotor to assure accurate angular positioning. The cam-rotor unit rotates loosely on shaft 376, the base of which is press fit into plate 324. The unit is retained by retaining element 377 secured to the top of the shaft. The rotor is preferably made of platinum cobalt or samarium cobalt (which may be plated with platinum to improve corrosion resistance). The rotor has ten permanently magnetic poles 374 of alternate polarity (Figs. 25-26). At any one angular position, the pole exposed on the top surface of the disk is opposite that of the one exposed on the bottom surface.

Below rotor 372 there are fixed in place four stator elements 378 each made of a material that is magnetically soft and permeable, and that is resistant to corrosion in the presence of cerebrospinal fluid, which contains chlorides. Preferred materials include magnetic stainless steel alloys and alloys of nickel, iron, and molybdenum or cobalt. As shown in Fig. 8, the stator elements are embedded in a plastic member 380, which is fixed to plate 324 by means of shaft 376. The stator elements are shaped so that the portion of each lying beneath the rotor matches the size of permanent magnets 374. The portions of the stator elements lying radially beyond the rotor are sized to match the area beneath the rotor so that the boundary between poles, when the stator is magnetized, is at the perimeter of the rotor.

### Operation

In operation the shunt valve assembly is surgically implanted in a patient following well-known procedures. Before implantation the pressure of adjustable valve 314 can be set to the desired level according to the circumstances of the case. For instance, it can be set approximately equal to the patient's pre-operative ventricular CSF pressure so that no immediate pressure change occurs as a result of the operation. After the patient has recovered from the trauma of the operation, the pressure is adjusted downwardly to the desired level. In the case of normal-pressure hydrocephalus, the pressure is lowered to a level sufficient to initiate shrinkage of the cerebral ventricle. Further adjustments in pressure can be made at subsequent times, as necessary. In the typical treatment of normal-pressure hydrocephalus, the pressure would be adjusted upwardly after sufficient shrinkage of the ventricle has occurred in order to stabilize ventricle size.

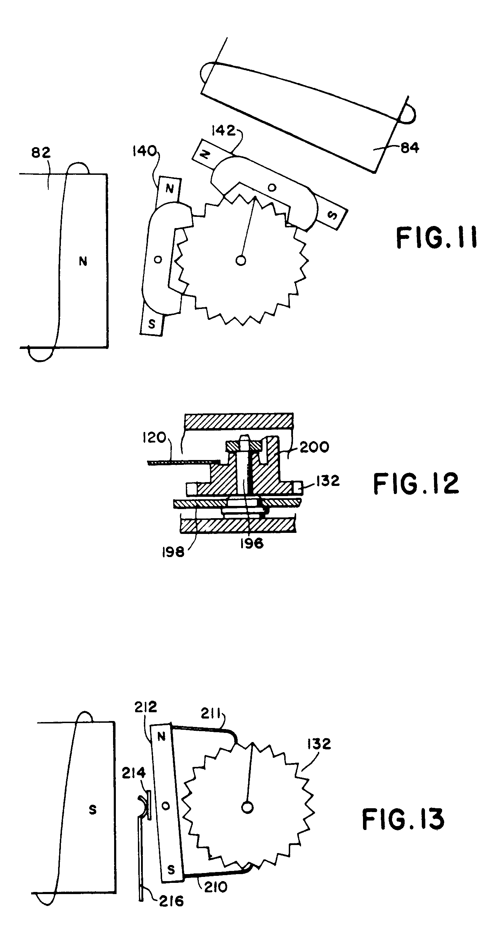

In children at the beginning of the treatment, the pressure should be lowered to a level inversely proportional to the ventricle size to reduce stress on the brain parenchyma (see Fig. 13 of Hakim et al., "The Physics of the Cranial Cavity", Surg. Neurol., Vol. 5, March 1976), and as the ventricle decreases in size the pressure of the valve should be increased, so when the ventricle attains normal size the intraventricular pressure is again normal, thereby avoiding development in the patient of a slit-ventricle condition. Also in cases of normal-pressure hydrocephalus, sometimes in spite of a low-pressure valve the patient does not improve and the ventricle size remains unchanged, making the surgeon think he is dealing with a case of brain atrophy. But by further changing the valve pressure to a lower one, the ventricle decreases in size and the patient immediately starts to improve. In elderly persons and in long standing cases of normal-pressure hydrocephalus, it has been found that the intraventricular pressure needs to be lowered more than in young people and in hydrocephalus of short duration.

Another advantage of the invention arises in the procedure for determining when an implanted shunt valve can safely be removed from a patient, i.e., determining whether the patient is still dependent on the valve for drainage of excess cerebrospinal fluid. The conventional technique for making that determination has been to temporarily pinch closed the tube downstream of the valve and observe the patient for symptoms (e.g., slight headache) indicative of valve dependency. In the absence of symptoms the valve can be removed. With the invention it is unnecessary to stop flow entirely. A safer procedure can be followed. Valve pressure is raised, slightly at first, more so later for confirmation, using the adjustment mechanism.

Turning to the embodiment of Figs. 1-5, valve pressure adjustments are made by applying a pulsed magnetic field to the vicinity of the shunt valve as shown diagrammatically in Fig. 5. A valve adjustment element 80 is applied over adjustable valve 14 in the orientation shown. The adjustment element 80 contains two electromagnets 82, 84, which are separately controlled by an external control device, shown diagrammatically at 86. The adjustment element has a marking (such as an arrow pointing in the direction of CSF flow) on its exterior to assure that it is applied to the valve in the correct orientation. Control device 86 has controls that permit the operator to choose the direction of adjustment, i.e., higher or lower pressure, and the amount of adjustment. The direction of pressure adjustment is determined by which of the two magnets 82, 84 is energized. The amount of adjustment is determined by the number of pulses applied to the selected magnet. Each application of a pulse causes magnetic arm 48 to move toward the interior face 60 of the valve housing, and then back to its neutral position, as shown in Figs. 3a and 3b, which illustrate the two directions of movement of the arm. In Fig. 3a, magnet 84 is energized, and the arm is moved between its neutral position and the extreme (upper in the figure) position shown. Travel of the arm is stopped by contact of the magnet 54 with interior surface 60 (Fig. 5) of the valve housing. In Fig. 3b movement in the opposite direction is shown. Each cycle of arm 52 causes escapement element 62 to engage a tooth of gear 46 and thereby rotate the gear through a small angle. Arm 52 bends slightly during the cycle to permit the escapement element to so engage. Torsion spring 56 provides a restoring torque to the arm. Each electrical pulse supplied to the magnets has a shape selected to move arm 52 slowly out of and back into its neutral position, so that there is little or no overtravel of the arm beyond neutral, thus avoiding undesired reverse rotation of gear 46.

The embodiment of Figs. 6-10 is surgically implanted in the same manner as the first-described embodiment.

Adjustments are made to the popping pressure using an adjustment element and control device of the type shown in Fig. 5 for the first embodiment. A magnetic field which alternates polarity with each pulse is applied from one or the other side of the valve assembly. Both magnets 140, 142 are influenced by the field, but the magnet that is closest to the source of the field dominates the other due to the fact that the strength of a magnetic field decreases as the square of the distance from its source. Thus, for purposes of understanding operation of the valve, it can be assumed that only the magnet closest to the source of the field is driven. The time-variation in the strength of the magnetic field is selected to cause the magnet, and the escapement element connected to it, to rotate back and forth sufficiently to cause gear 132 to rotate by the angle corresponding to one tooth for each full cycle of the escapement element.

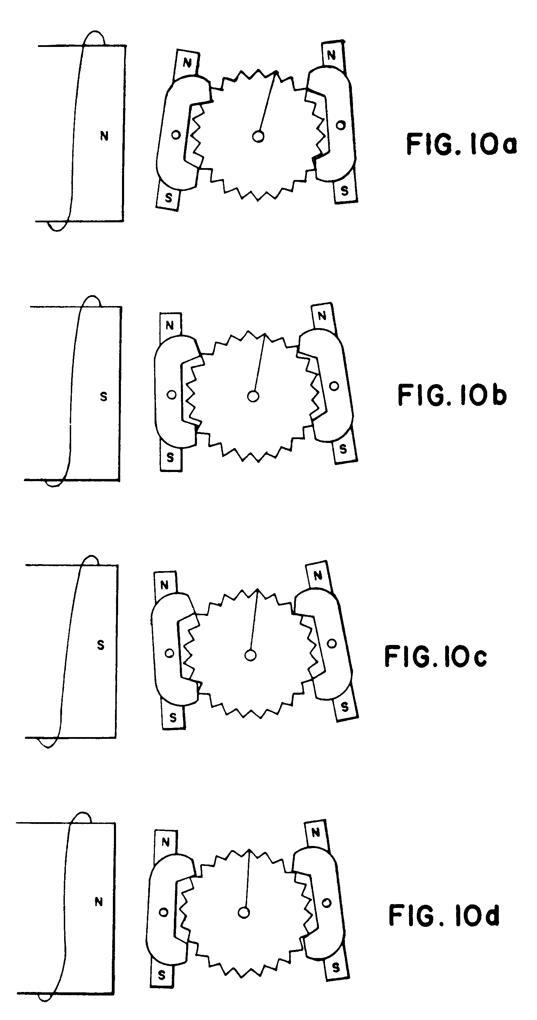

Shown in Figs. 10a-10d are diagrammatic views of four stages during one cycle of the escapement element. When the electromagnet is energized with north polarity, this causes the escapement magnet to rotate; the south pole of the escapement magnet is attracted toward the electromagnet, and the north pole is repelled away therefrom. In the figures, the gear has more teeth than in the preferred embodiment, and the escapement elements are shown diagrammatically.

Rotation of gear 132 causes screw 118 and, in turn, one end of spring arm 120, to move upwardly or downwardly. This, in turn, changes the force applied by spring 116 to ball 110, and thus the popping pressure at which fluid pressure on the ball will lift it away from the valve seat. The advantages of using the type of spring shown are that a very low K-factor (spring rate) can be provided at the ball (and thus little change in popping pressure for change in flow rate) and the bias force on the ball is less sensitive to changes in the height of screw 118 than would be another type of spring (thus allowing finer adjustment of popping pressure). One full revolution of the gear comprises the full extent of ordinary adjustment of the popping pressure.

The embodiment of Figs. 17-32 is surgically implanted in the same manner as the first-described embodiment.

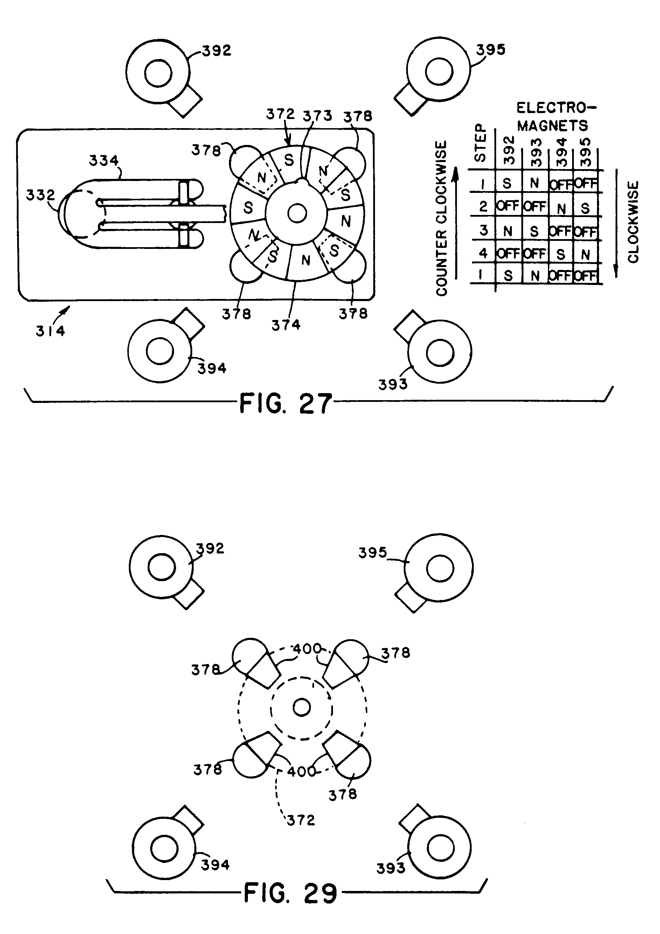

Valve pressure adjustments are made by applying a pulsed magnetic field to the vicinity of the shunt valve as shown diagrammatically in Figs. 27-29. A valve adjustment element 390 is applied over adjustable valve 314 in the orientation shown. The adjustment element contains four electromagnets 392, 393, 394, 395, which are separately controlled by an external control device, shown diagrammatically at 396. Adjustment element 390 has a marking (such as an arrow pointing in the direction of CSF flow) on its exterior to assure that it is applied to the valve in the correct orientation, and it has a groove 398 in its bottom surface sized to fit over the protrusion in the scalp, at the site of the implanted valve. The groove is narrowed at one end 399 to enable correct longitudinal alignment relative to the adjustable valve 314.

Control device 396 has input keys, which the operator uses to select one of 18 possible desired pressures (from 20 to 190 mm H₂O) and a pressure display.

Each of electromagnets 392, 393, 394, 395 can be energized to have either the north or south polarity facing the stator elements, or each can remain off altogether. Movement of rotor 372, in the desired direction and through the desired angle, is achieved by energizing the electromagnets in the sequence shown in the table in Fig. 27. For example, clockwise motion is achieved by first energizing electromagnets 392, 393 to south and north polarities, respectively, and leaving electromagnets 394, 395 off. In the next step electromagnets 392, 393 are left off, and electromagnets 394, 395 are energized to north and south polarities, respectively. The sequence repeats itself after the fourth step. Rotor 372 is shown in Fig. 27 in the position reached after the first step (the polarities of the rotor magnets are those on the bottom surface). If the magnetic field provided by the electromagnets is described by a vector pointing from the south to the north pole of the energized magnets, then it can be seen that the sequence prescribed for causing rotor 372 to rotate clockwise (down the table in Fig. 27) amounts to rotating the field vector in the counterclockwise direction (opposite that of the rotor), in 90° steps.

Electromagnets 392, 393, 394, 395 are positioned 90° apart and spaced equal radial distances from a central axis. When adjustment device 390 is installed properly over valve 314, the central axis of the electromagnets is coincident with the axis of rotation of rotor 372, and each electromagnet is aligned at the same angular position as one stator element 378. It is not, however, necessary that this alignment be exact. The invention is tolerant of alignment errors, which are unavoidable owing to the inability of the user to see rotor 372 or stator elements 378 and to the small size of those elements relative to the size of the external electromagnets.

The magnetic polarization induced in the stator elements 378 as the result of energizing the electromagnets is diagrammatically illustrated in Fig. 29. The two stator elements along the axis connecting the two energized electromagnets are polarized in the radial direction, so that the boundary between the poles lies roughly at the peripheral edge of disk rotor 372. The radially inner portions of these two stator elements, the portions lying beneath rotor 372, have the opposite polarity of the portions lying outside. By contrast, the stator elements along the other axis are polarized so that the boundary between poles lies along the radial direction. Both poles extend beneath the rotor 372. This pattern of polarization will result even if there is substantial error in the orientation of the electromagnets.

Movement of rotor 372 is influenced predominantly by the stator regions 400 (shown in dashed lines in Fig. 29) lying beneath the rotor, as it is those portions that are closest to the permanent magnets 374 of the rotor. Accordingly, the part of the stator elements with uniform polarity dominate over those with split polarity. This phenomenon could be emphasized by making the stator elements of a magnetically anisotropic material so that the magnetization induced by the external electromagnets is strongest along the radial axis of the corresponding stator elements.

The number of magnetic poles 374 is selected so that when one pair of radially opposite stator elements 378 is aligned with one pair of magnetic poles 374 (as are the upper left and lower right stator elements in Fig. 27) the other two stator elements (the upper right and lower left in Fig. 27) are each staggered halfway between two of the poles 374. In operation, control device 396 energizes the electromagnets closest to the pair of stators staggered between two magnets, thereby causing the rotor to move through an angle corresponding to one half the width of a magnetic pole 374.

In the preferred embodiment there are ten magnetic poles on each side of the disk, and thus twenty angular increments in one full revolution (i.e., each step is one twentieth of 360°, or 18°). Only eighteen of these increments are used, corresponding to the eighteen detented steps along the staircase surface of cam 368 (the other two increments are occupied by the detent wall 370 of the cam).

After a pressure is prescribed on control device 396, an enter key is pressed. That initiates a sequence of eighteen steps in the direction of lower pressure settings, counterclockwise rotation of rotor 372. This assures that the cam is returned to a position wherein spring arm 364 is at the lowest step on the cam staircase. If fewer than eighteen steps are actually needed to bring the cam to this position (as will most often be the case), the detent wall provided by element 370 of the cam prevents further rotation. After the eighteen-step resetting sequence is complete, the rotor is moved clockwise by the number of steps corresponding to the prescribed pressure.

Various variations can be made to the embodiment of Figs. 17-32.

A magnetically anisotropic material could be used for the stator elements, with the strongest axis of magnetization oriented along the radial direction. Such anisotropy could also be achieved mechanically by splitting each stator element along the radial direction into two or more segments.

A variable reluctance or hybrid rotor could replace permanent-magnet rotor 372.

Linear movements within an implanted device could be achieved following the invention by providing a linearly-moving element as the rotor and by placing stator elements along the path of the linearly-moving rotor.

A rotor with fewer poles could replace the ten-pole rotor of the preferred embodiment, particularly where fine angular precision is not required (e.g., in a pump a simple two-pole rotor might suffice). Strong permanent magnets could be used to apply the external field (e.g., in the two-pole rotor of the pump application just described).

Electrical wire could be wrapped around the implanted stator elements forming a coil so that an electrical current is induced therein by the externally-pulsed magnetic field; the electrical current would in turn magnetize the stator elements if the coil circuit is closed by a resistor or a capacitor.

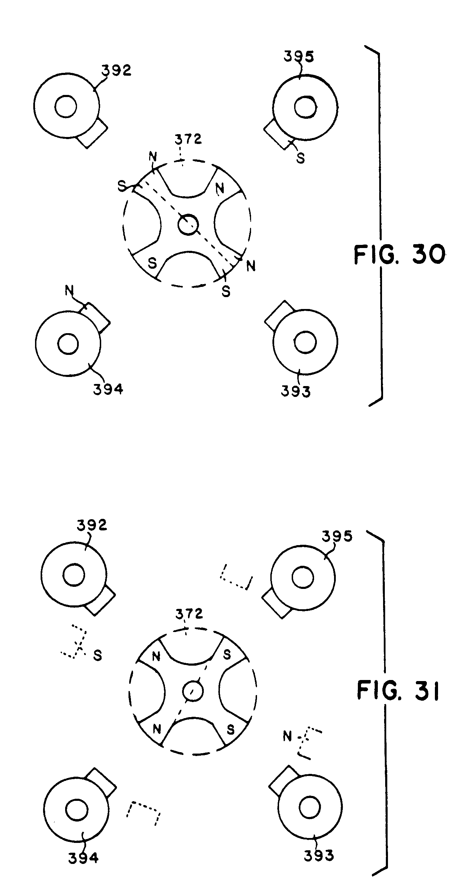

A single-piece stator element, e.g., with four lobes as shown in Fig. 30, could replace the stator elements of the preferred embodiment. An advantage of using a single-piece stator element is that the stator can lie entirely inside of the outer perimeter of the rotor, and thus provide for a more compact implanted unit. This is because, when magnetized, as shown in Fig. 30, the dominating lobes are all of one polarity, rather than split radially into two polarity regions as in the preferred embodiment. Thus the entire lobe, not just the inner half, can be influential in moving the rotor. The disadvantages of the single-piece stator is its lower tolerance to errors in alignment of the external magnetic field. The reduced tolerance for misalignment can be understood by reference to Fig. 31, in which the external field has been rotated sufficiently to cause the lobes that were split into two poles to now be entirely within the region of one pole. As a result all four lobes have nearly equal influence on the rotor, and it is not possible to move the rotor.

A presently even more preferred embodiment is one having a rotor 372 with six poles rather than ten (as shown in the figures). Such a rotor provides twelve steps of 30° for one full revolution. Eleven of these can be used to provide eleven different pressure settings (each differing by 15 mm H₂O) from 30 to 180 mm H₂O. An advantage of six poles is that with the four-stator-element configuration (Fig. 27) greater torque is available using six rather than ten poles. This results because all four stator elements, when magnetized by the external field, generate a torque in the same direction. In the ten-pole embodiment (Fig. 27), this is not the case. The torque generated by the stator elements with split polarity (upper right and lower left in Fig. 27) is opposite, though weaker than, that generated by the stator elements with uniform polarity. It is instructive to note, however, that this difference between the six and ten-pole embodiments is just the opposite if a one-piece, four-lobed stator is used. In that case, the split polarity stator elements generate opposing torque with the six-pole rotor and not with the ten-pole one. Also, if a ten-pole rotor is used with separate stator elements, torque can be increased by using anisotropic material for the stator elements, as that will increase the dominance of the uniform polarity stator elements over the split polarity ones.

Another embodiment is shown in Fig. 11. One of magnets 140, 142 (and the associated escapement element) is positioned approximately 90° around the gear from the other magnet so as to be approximately normal to the other magnet. The valve adjustment element for use with this valve is modified so that external electromagnets 82, 84 are also positioned at roughly 90° to one another (as suggested in Fig. 11). The 90° orientation helps increase the dominance that one of magnets 140, 142 has over the other when only one of external electromagnets 82, 84 is energized.

Another variation that is preferred is the use of a cam 200, as shown in Fig. 12, instead of screw 118, to adjust the height of spring arm 120 in response to rotation of gear 132. Others are to use fewer teeth (e.g., fourteen) on gear 132, to provide a stop for gear 132 (e.g., a pin extending from the gear and abutting a tab) to stop its rotation at positions corresponding to the minimum and maximum popping pressures, and to mount gear 132 and the escapement elements on stub shafts 196 pressed through plate 198 (equivalent to plate 106 of Fig. 7). The stop is useful during adjustment; e.g., gear 132 can be rotated counterclockwise until it reaches the stop and then a desired number of clockwise rotational increments can be prescribed therefrom, to provide a repeatable, accurate setting of popping pressure.

Instead of using two escapement elements of the type shown in Figs. 10a-10d, it may be preferred to use only one escapement element of the type shown in Fig. 13. The blunt teeth of the Fig. 10 escapement elements have been replaced by thin, flexible elements 210, 211, attached at either end of single magnet 212. This construction is better able to accommodate debris that may collect in the valve, as thin elements 210, 211 are better able than blunt teeth to pierce through any debris that might collect between the gear teeth. Each pulse of an externally-applied magnetic field has the effect of momentarily, rotating magnet 212 against the torsional centering spring formed by plate 214 and spring 216, and thereby rotating gear 132 by the angular separation of its teeth. The element doing the pushing (e.g., element 210 in Fig. 13) during the movement remains relatively rigid, and thus overcomes the much smaller retarding force of the other element (e.g., element 211 in the figure), which tends to bend out of the way of the gear teeth. At the end of each pulse, the centering spring 216 brings magnet 212 and elements 210, 211 back to the rest position.

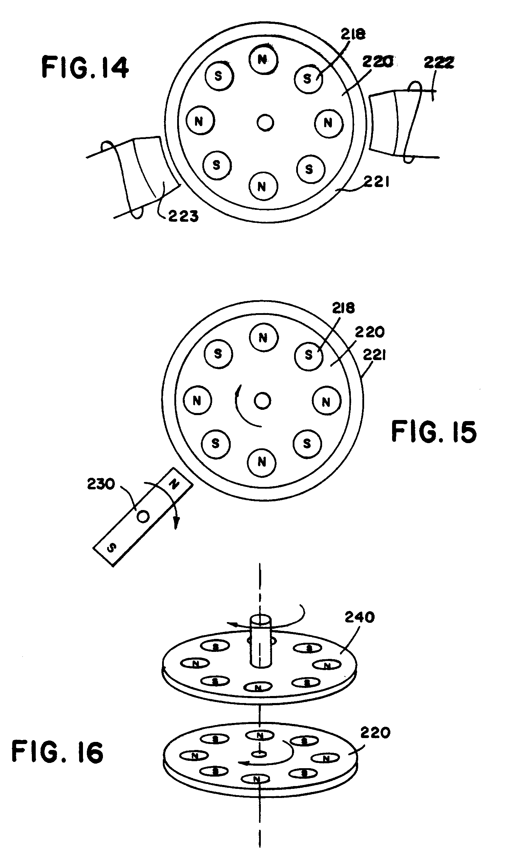

Another embodiment uses a very small stepper motor similar to the type used in electronic watches in place of the disclosed mechanisms for rotating the spring adjustment screw; the rotor of the stepping motor would reside in the valve, and the stator would be placed in the external adjustment element. (This contrasts with the embodiment of Figs. 17-32, wherein part of the stator resides in the valve.) Such an arrangement is shown in Fig. 14. A plurality of alternate polarity permanent magnets 218 are formed on disk 220 (samarium-cobalt); one pole of each magnet 218 is on the top surface of the disk; the other pole is on the bottom surface. The disk 220 is attached to a wheel 221, that replaces gear 132, to form the rotor portion of the stepper motor. External electromagnets 222, 223 are positioned as shown in Fig. 14 with respect to disk 220; when one external electromagnet 222 is aligned with one of magnets 218, the other external electromagnet 223 is staggered halfway between two of magnets 218. In operation, electromagnets 222, 223 are pulsed alternately, so that the electromagnet pulsed is the one staggered between two of magnets 218. The direction of rotation of the disk 220 will be determined by the polarity of this magnet. This embodiment generally requires a large rotor and accurate positioning of the external stator.

Another variation is to use a pair of permanent magnets attached at diametrically opposed locations on a wheel that would replace the toothed gear of the disclosed embodiments. An external horseshoe magnet would be aligned with the magnets on the wheel and would then be turned to make a pressure adjustment.

Another variation would be to provide wheel 221, disk 220, and circular array of permanent magnets 218 described in connection with Fig. 14, but to rotate wheel 221 by rotating an external magnet 230 positioned as shown in Fig. 15. Each revolution of adjusting magnet 230 advances wheel 221 by the angular separation between two of magnets 218. The rotating magnet 230 could be replaced by other mechanisms designed to move a magnetic field along a generally circumferential direction past magnets 218; e.g., a coil magnet could be repetitively swept past disk 220 by action of a solenoid and return spring, with the coil magnet being energized only when moving in one direction.

Another variation is shown in Fig. 16. The external adjusting device is a disk 240 (samarium-cobalt) having the same array of permanent magnets as on disk 220 inside the valve. Identity of the arrays makes possible turning the internal wheel in synchronism with rotation of the external disk.

## Claims

1. For venting cerebrospinal fluid in the treatment of hydrocephalus or for shunting other body fluids, a surgically-implantable shunt valve (14; 100; 314) comprising: a housing (22; 102;104; 322) constructed of a surgically-implantable material; an inlet (360) and outlet (362) chamber within said housing, said housing including inlet and outlet ports communicating with said inlet and outlet chambers respectively for connecting said inlet and outlet chambers to external catheters or other fluid conduits; an aperture (26; 108; 326,328) communicating between said chambers, said aperture having a circular periphery forming a circular valve seat; a valve member (28; 110; 332) of diameter larger than said circular valve seat such that it fits into said circular valve seat so as to seal it; and spring means (30; 116; 334) for biasing said valve member against the circular valve seat, said spring means being adapted to keep said aperture closed until fluid pressure in said inlet chamber exceeds a preselected popping pressure and adapted to open said aperture when said popping pressure is exceeded so as to vent fluid through said aperture into said outlet chamber; said valve being characterised in further comprising incremental magnetic adjustment means adapted to increase or decrease the amount of said bias in finite increments in response to pulses of magnetic field applied from outside a body in which the valve is implanted, so as thereby to increase or decrease said popping pressure in finite increments, said incremental magnetic adjusting means comprising a primary member (46; 118; 132; 366,372) adapted to co-operate with said spring means so as to increase or decrease said bias when said primary member is moved, said primary member not itself being susceptible to movement by said external magnetic field, and a secondary member (48; 136,138,140,142; 212; 378,380) susceptible to said applied magnetic field and mounted and positioned with respect to said primary member so as to be capable of movement relative to said primary member and so as to cause no more than an incremental movement of said primary member for each pulse of said magnetic field.

2. A valve according to Claim 1, further characterised in that stop means (370) are provided for stopping movement of said primary member at a position corresponding to a known popping pressure, to thereby permit pressure adjustments to be made by first causing said primary member to move against said stop and then incrementally moving said primary member away from said stop.

3. A valve according to Claims 1 or 2, further characterised in that said primary member comprises screw or cam means (118; 200; 366) rotatable to increase or decrease said bias.

4. A valve according to Claim 3, further characterised in that said primary member includes a toothed gear (46; 132) mounted for rotation and coupled to said screw or cam means, and said secondary member comprises an escapement element (62; 136,138; 210,211) engaging the teeth of said gear and a permanent magnet (54; 140,142; 212) for moving said escapement element in response to said applied pulsed magnetic field to thereby rotate said toothed gear.

5. A valve according to Claim 4, further characterised in that said escapement element (62) is mounted on an arm (52), in that said arm is pivoted about an axis, and in that a ferromagnetic or permanently magnetic material (54) is attached to said arm at a radial location further from said pivot than said escapement element.

6. A valve according to Claim 5, further characterised in that spring means (56) are provided for returning said escapement element and arm to a neutral position.

7. A valve according to Claim 6, further characterised in that said arm (52), escapement element (62), gear (46) and spring means (56) are adapted so that application of a varying magnetic field that rises and then decays has the effect of moving said arm through a cycle consisting of movement in one direction from said neutral position to a stop position and then back again and so that as the result of said movement said gear rotates through a finite angle, said spring portion is raised or lowered a finite distance, and said popping pressure is varied by a finite movement.

8. A valve according to Claim 7, further characterised in that more than ten such cycles are required to raise said popping pressure from its minimum to its maximum.

9. A valve according to Claim 4, further characterised in that there are two said escapement elements (136,138; 210,211) engaging said gear (132) at different angular positions about the gear.

10. A valve according to Claim 9, further characterised in that the angular locations of the axes of rotation of said escapement elements are separated angularly around said gear by a whole number of gear teeth plus one half of a tooth.

11. A valve according to Claims 9 or 10, further characterised in that said two escapement elements are positioned about 180° apart around said gear.

12. A valve according to Claim 9 or 10, further characterised in that said two escapement elements are positioned about 90° apart around said gear.

13. A valve according to any of Claims 9 to 12, further characterised in that said two escapement elements (136,138) are each connected to a permanent magnet (140,142) and each escapement/magnet pair is pivoted about an axis (144) passing between the two poles of the magnet.

14. A valve according to both Claim 11 and Claim 13, further characterised in that for each said magnet the line connecting the poles of said magnet is generally parallel to said line on the other of said magnets.

15. A valve according to both Claim 12 and Claim 13, further characterised in that for each said magnet the line connecting the poles of said magnet is generally normal to said line on the other of said magnets.

16. A valve according to any of Claims 9 to 15, further characterised in that said escapement elements are positioned so that an external magnetic field can be applied in a first location such that the field provides a stronger turning moment on one said escapement than the other, and so that if said external field is applied in a second location a stronger turning moment will be provided to the other of said escapements.

17. A valve according to any preceding Claim, further characterised in that movement of said primary member is translated into movement of a portion of said spring means, and in that said spring means comprises a three-arm spring having two flanking arms (36,38; 122; 340,342) and a central arm (32; 120; 338), one end of said central arm (34; 336) being positioned to bias said valve member against said circular valve seat, and the other end of said central arm being said portion of said spring means.

18. A valve according to Claim 17, further characterised in that the ends of said flanking arms (122; 340,342) are retained by a yoke (126; 346) that extends over the upper surfaces of said ends to retain said ends against upward movement.

19. A valve according to Claim 18, further characterised in that said yoke (126; 346) extends from below said ends of said flanking arms (122; 340,342), past the inward edges (123) of said arms, and over said ends, thereby to retain said ends against sideward as well as upward movement.

20. A valve according to Claim 19, further characterised in that said yoke is U-shaped so as to pass between said central arm and said flanking arms.

21. A valve according to Claims 19 or 20, further characterised in that each of said ends of said flanking arms (122) have a notch (123) receiving that portion of said yoke passing said inward edges of said arms, thereby to retain said spring against longitudinal movement as well as upward and sideward movement.

22. A valve according to any of Claims 18 to 21, further characterised in that said yoke is not fastened to said ends of said flanking arms but fits loosely thereover.

23. A valve according to any of Claims 17 to 22, as appendant to Claim 3, further characterised in that said cam or screw means comprises a screw extending through said spring portion (Fig. 2).

24. A valve according to any of Claims 17 to 22, further characterised in that said cam or screw means comprises a cam surface (200) adapted to lift said spring portion (120) as said gear (132) is rotated.

25. A valve according to any of Claims 1 to 16, further characterised in that said primary member comprises the rotor (372) of a stepping motor, and said secondary member comprises a plurality of stator elements (378) comprised of magnetically soft and permeable material shaped and positioned with respect to said rotor so that when magnetized under the influence of said external magnetic field the said stator elements strengthen and orient the magnetic field in their vicinities so as to cause movement of said rotor; and in that movement of said primary member is translated into movement of a portion of said spring means (364) by a cam (366) in the shape of a circular staircase with a portion of said spring means in contact with said staircase, wherein rotation of said rotor causes rotation of said cam and a change in the position of said spring portion along said staircase.

26. A valve according to any preceding Claim, further characterised in that said spring means comprises a spring having a low spring rate to make said popping pressure substantially independent of changes in fluid flow rate, and in that said incremental magnetic adjustment means is effective to increase or decrease said bias without substantially affecting the spring rate of said spring.

27. A valve according to Claim 4, further characterised in that said escapement element has blunt teeth of a shape that mates with the teeth of said gear.

28. A valve according to Claim 4, further characterised in that said escapement element has teeth in the form of long, thin elements (210,211) adapted for piercing through debris collected between the teeth of said gear.

29. A valve according to any of Claims 4 to 16, further characterised in that said escapement element(s) has (have) two teeth.

30. For venting cerebrospinal fluid in the treatment of hydrocephalus or for shunting other body fluids, a surgically-implantable shunt valve (314) comprising: a housing (322) constructed of a surgically-implantable material; an inlet (360) and outlet (362) chamber within said housing, said housing including inlet and outlet ports communicating with said inlet and outlet chambers respectively for connecting said inlet and outlet chambers to external catheters or other fluid conduits; an aperture (326) communicating between said chambers, said aperture having a circular periphery forming a circular valve seat (328); a valve member (332) of diameter larger than said circular valve seat such that it fits into said circular valve seat so as to seal it; and spring means (334) for biasing said valve member against the circular valve seat, said spring means being adapted to keep said aperture closed until fluid pressure in said inlet chamber exceeds a preselected popping pressure and adapted to open said aperture when said popping pressure is exceeded so as to vent fluid through said aperture into said outlet chamber; said valve being characterised in further comprising incremental magnetic adjustment means adapted to increase or decrease the amount of said bias in finite increments in response to pulses of an applied magnetic field so as thereby to increase or decrease said popping pressure in finite increments, said incremental magnetic adjusting means comprising an element (372) adapted to rotate in response to an external magnetic field, and a cam (366) in the shape of a circular staircase, said spring means including an arm (364) supported on said staircase.

31. Apparatus for venting cerebrospinal fluid in the treatment of hydrocephalus or for shunting other body fluids, the apparatus being characterised in comprising: a surgically implantable shunt valve as defined in any preceding Claim; and adjustment apparatus (80; 390) adapted to provide a pulsed magnetic field for adjustment of said shunt valve, said adjustment apparatus comprising an electromagnet (82,84; 392,393,394,395) and means adapted for orienting said apparatus and thereby said magnet in a predetermined orientation with respect to said valve so that energization of said electromagnet will have a predictable effect on the popping pressure of said valve.

32. Apparatus according to Claim 31, further characterised in that said orienting means comprises a recess shaped to fit over the protrusion which would be formed on the skin surface of a patient by said valve when implanted thereunder.

33. Apparatus according to Claim 32, further characterised in that said orienting means comprises a marking for indicating the direction of fluid flow so that the user can orient said adjustment apparatus so that said arrow points in the direction of fluid flow through said valve.

34. Apparatus according to any of Claims 31, 32 or 33, further characterised in that said adjustment apparatus comprises two electromagnets, each separately energizeable and each oriented at an angle to the other so that upward adjustment of said popping pressure may be achieved by energizing one of said electromagnets and so that downward adjustment may be achieved by energizing the other of said electromagnets.

## Patentansprüche

1. Chirurgisch implantierbares Nebenschlußventil (shunt valve) (14; 100; 314) für die Drainage zerebrospinaler Flüssigkeit in der Behandlung von Hydrocephalus oder zur Ableitung anderer Körperflüssigkeiten, umfassend: ein Gehäuse (22; 102; 104; 322) aus chirurgisch implantierbarem Material; eine Eingangs- (360) und eine Ausgangskammer (362) innerhalb dieses Gehäuses, wobei das Gehäuse Eingangs- und Ausgangsöffnungen umfaßt, die mit der Eingangs- bzw. Auzgangskammer kommunizieren, um die Eingangs- bzw. Ausgangskammer mit externen Kathetern oder anderen Flüssigkeitskanälen zu verbinden; eine Öffnung (26; 108; 326,328) zwischen den Kammern, wobei diese Öffnung kreisförmig ist und einen kreisförmigen Ventilsitz bildet; ein Ventilglied (28; 110; 332) mit einem Durchmesser, der größer ist als der kreisförmige Ventilsitz, derart, daß es in den Ventilsitz hineinpaßt und ihn verschließt; und ein Federelement (30; 116; 334), um das Ventilglied mit Vorspannung gegen den kreisförmigen Ventilsitz zu pressen, wobei das Federelement dafür ausgelegt ist, die Öffnung geschlossen zu halten, bis der Flüssigkeitsdruck in der Eingangskammer einen vorher festgelegten Ventilöffnungsdruck übersteigt, und dafür ausgelegt ist, das Ventil zu öffnen, wenn der Ventilöffnungsdruck überschritten wird, und so Flüssigkeit durch die Öffnung in die Ausgangskammer abzuleiten;
**dadurch gekennzeichnet,**
daß das Ventil weiterhin eine magnetische inkrementale Regelvorrichtung umfaßt, die dafür ausgelegt ist, den Betrag der Vorspannung in endlichen Schritten zu erhöhen oder zu senken entsprechend Magnetfeldimpulsen, die von außerhalb eines Körpers, in den das Ventil implantiert ist, appliziert werden, derart, daß dadurch der Ventilöffnungsdruck in endlichen Schritten erhöht oder gesenkt wird, wobei die magnetische inkrementale Regelvorrichtung ein Primärglied (46; 118; 132; 366,372) umfaßt, das mit dem Federelement derart zusammenwirkt, daß es die Federspannung erhöht oder senkt, wenn das Primärglied bewegt wird, wobei das Primärglied selbst nicht durch das externe Magnetfeld bewegbar ist, und ein Sekundärglied (48; 136,138,140,142; 212; 378;380), das auf das applizierte Magnetfeld reagiert und hinsichtlich des Primärgliedes so befestigt und ausgerichtet ist, daß es relativ zu dem Primärglied eine Bewegung ausüben kann, derart, daß es nicht mehr als eine inkrementierende Bewegung des Primärgliedes bei jedem Impuls des Magnetfeldes verursacht.

2. Ventil nach Anspruch 1, dadurch gekennzeichnet, daß Anschläge (370) vorgesehen sind, um die Bewegung des Primärgliedes an einer Position zu stoppen, die einem bekannten Ventilöffnungsdruck entspricht, und dadurch Druckeinstellungen in der Weise zu ermöglichen, daß zunächst das Primärglied gegen den Anschlag geführt und anschließend schrittweise von dem Anschlag wegbewegt wird.

3. Ventil nach Ansprüchen 1 oder 2, dadurch gekennzeichnet, daß das Primärglied Schrauben oder Nocken (118; 200; 366) umfaßt, durch deren Drehung die Federspannung erhöht oder gesenkt werden kann.

4. Ventil nach Anspruch 3, dadurch gekennzeichnet, daß das Primärglied ein drehbeweglich befestigtes Zahnrad umfaßt (46; 132), das mit der Schraube oder Nocke gekoppelt ist, und daß das Sekundärglied ein Hemmungselement (62; 136,138; 210,211), das mit den Zähnen des Zahnrades in Eingriff steht, und einen Permanentmagneten (54; 140,142; 212) umfaßt, der das Hemmungselement entsprechend dem ausgeübten Magnetfeldtakt bewegt und dadurch das Zahnrad dreht.

5. Ventil nach Anspruch 4, dadurch gekennzeichnet, daß das Hemmungselement (62) auf einem Arm (52) befestigt ist, daß der Arm um eine Achse drehbar ist und daß an dem Arm ein ferromagnetisches oder permanentmagnetisches Material (54) angebracht ist, in einer radialen Position, die von der Drehachse weiter entfernt ist als das Hemmungselement.

6. Ventil nach Anspruch 5, dadurch gekennzeichnet, daß Federvorrichtungen (56) vorgesehen sind, um das Hemmungselement und den Arm in eine neutrale Position zurückzubewegen.

7. Ventil nach Anspruch 6, dadurch gekennzeichnet, daß der Arm (52), das Hemmungselement (62), das Zahnrad (46) und die Federvorrichtung (56) so ausgelegt sind, daß die Einwirkung eines veränderlichen magnetischen Feldes, das stärker wird und dann abnimmt, den Arm zyklisch bewegt, derart, daß diese Bewegung zunächst in der Richtung von der neutralen Position zu einer Halteposition und dann wieder zurück führt, so daß als Ergebnis dieser Bewegung das Zahnrad um einen endlichen Winkelbetrag gedreht wird, wobei das Federelement um eine endliche Distanz erhöht oder gesenkt wird und der Ventilöffnungsdruck durch eine endliche Bewegung variiert wird.

8. Ventil nach Anspruch 7, dadurch gekennzeichnet, daß mehr als zehn solcher Zyklen erforderlich sind, um den Ventilöffnungsdruck von seinem Minimum auf sein Maximum zu erhöhen.

9. Ventil nach Anspruch 4, dadurch gekennzeichnet, daß zwei der Hemmungselemente (136,138; 210,211) mit dem Zahnrad in unterschiedlichen Winkelpositionen zu demselben in Eingriff stehen.

10. Ventil nach Anspruch 9, dadurch gekennzeichnet, daß die Winkelpositionen der Drehachsen der Hemmungselemente winkelig um eine ganzzahlige Anzahl von Zähnen plus einen halben Zahn um das Zahnrad beabstandet sind.

11. Ventil nach Anspruch 9 oder 10, dadurch gekennzeichnet, daß die zwei Hemmungselemente in einem Winkelabstand von 180° um das Zahnrad angeordnet sind.

12. Ventil nach Anspruch 9 oder 10, dadurch gekennzeichnet, daß die zwei Hemmungselemente in einem Winkelabstand von 90° um das Zahnrad angeordnet sind.

13. Ventil nach einem der Ansprüche 9 bis 12, dadurch gekennzeichnet, daß jedes der beiden Hemmungselemente (136,138) mit einem Permanentmagneten (140,142) verbunden ist und daß jedes Hemmungselement/Magnet-Paar um eine Achse (144) drehbar ist, die zwischen den beiden Polen des Magneten hindurchgeht.

14. Ventil nach Anspruch 11 und 13, dadurch gekennzeichnet, daß für jeden der beiden Magnete die Linie, die die Pole des Magneten miteinander verbindet, im allgemeinen parallel zu der gleichen Linie auf dem anderen Magneten ist.

15. Ventil nach Anspruch 12 und 13, dadurch gekennzeichnet, daß für jeden der beiden Magnete die Linie, die die Pole des Magneten miteinander verbindet, im allgemeinen senkrecht zu der gleichen Linie auf dem anderen Magneten ist.

16. Ventil nach einem der Ansprüche 9 bis 15, dadurch gekennzeichnet, daß die Hemmungselemente so angeordnet sind, daß ein externes magnetisches Feld in einer ersten Position so zur Einwirkung gebracht werden kann, daß das Feld auf eines der Hemmungselemente ein stärkeres Drehmoment ausübt als auf das andere, und weiterhin so angeordnet sind, daß, wenn das Magnetfeld in einer zweiten Position zur Einwirkung gebracht wird, ein stärkeres Drehmoment auf das andere der beiden Hemmungselemente ausgeübt wird.

17. Ventil nach einem der vorhergehenden Ansprüche, dadurch gekennzeichnet, daß die Bewegung des Primärgliedes in Bewegung eines Abschnittes des Federelements umgesetzt wird und daß das Federelement eine dreiarmige Feder mit zwei Seitenarmen (36,38; 122; 340,342) und einem mittleren Arm (32; 120; 338) aufweist, wobei ein Ende des mittleren Armes (34; 336) so angeordnet ist, daß es das Ventilglied gegen den kreisförmigen Ventilsitz preßt, und das andere Ende des mittleren Armes der oben genannte Abschnitt des Federelementes ist.

18. Ventil nach Anspruch 17, dadurch gekennzeichnet, daß die Endabschnitte der Seitenarme (122; 340,342) von einem jochartigen Bügel (126; 346) zurückgehalten werden, der sich über die oberen Oberflächen der Endabschnitte erstreckt, um die Endabschnitte gegen eine Aufwärtsbewegung zurückzuhalten.

19. Ventil nach Anspruch 18, dadurch gekennzeichnet, daß sich der Bügel (126; 346) von unterhalb der Endabschnitte der Seitenarme (122; 340,342) an den inneren Rändern (123) der Seitenarme vorbei über die Endabschnitte erstreckt, um dadurch die Endabschnitte sowohl gegen eine seitliche Bewegung als auch gegen eine Aufwärtsbewegung zu sperren.

20. Ventil nach Anspruch 19, dadurch gekennzeichnet, daß der Bügel U-förmig ausgebildet ist, um dadurch zwischen dem mittleren Arm und den Seitenarmen durchzupassen.

21. Ventil nach Anspruch 19 oder 20, dadurch gekennzeichnet, daß jeder der Endabschnitte der Seitenarme (122) eine Kerbe (123) zur Aufnahme des an den Innenrändern der Arme vorbeiführenden Abschnittes des Bügels hat, wodurch das Federelement sowohl gegen Längsbewegung als auch gegen seitliche und Querbewegung gesperrt wird.

22. Ventil nach einem der Ansprüche 18 bis 21, dadurch gekennzeichnet, daß der Bügel nicht an den Endabschnitten der Seitenarme befestigt ist, sondern lose darüber eingepaßt ist.

23. Ventil nach einem der Ansprüche 17 bis 22, als Ergänzung zu Anspruch 3, dadurch gekennzeichnet, daß die Nocken- oder Schraubenvorrichtungen eine Schraube umfassen, die sich durch den Abschnitt des Federelements erstreckt (Fig. 2).

24. Ventil nach einem der Ansprüche 17 bis 22, dadurch gekennzeichnet, daß die Nocken- oder Schraubenvorrichtungen eine Nockenoberfläche (200) aufweisen, die dafür ausgelegt ist, den Federelementabschnitt (120) anzuheben, wenn das Zahnrad (132) gedreht wird.

25. Ventil nach einem der Ansprüche 1 bis 16, dadurch gekennzeichnet, daß das Primärglied den Rotor (372) eines Schrittmotors umfaßt und daß das Sekundärglied eine Vielzahl von aus magnetisch weichem und durchlässigem Material bestehenden Statorelementen (378) umfaßt, die im Hinblick auf den Rotor derart geformt und positioniert sind, daß die Statorelemente, wenn sie unter dem Einfluß des externen magnetischen Feldes magnetisiert werden, das magnetische Feld in ihrer Nähe verstärken und ausrichten und damit den Rotor in Bewegung versetzen; und daß die Bewegung des Primärgliedes durch eine Nocke (366) in Gestalt einer kreisförmigen Treppe in Bewegung eines Abschnittes des Federelementes (364) umgesetzt wird, wobei ein Abschnitt des Federelementes die Treppe berührt und eine Drehung des Rotors eine Drehung der Nocke und eine Änderung in der Position des Federelementabschnittes entlang der Treppe verursacht.

26. Ventil nach einem der vorhergehenden Ansprüche, dadurch gekennzeichnet, daß das Federelement eine Feder umfaßt, die eine niedrige Federkonstante hat, wodurch der Ventilöffnungsdruck im wesentlichen unabhängig von Änderungen in der Durchflußrate der Flüssigkeit wird, und daß die magnetische inkrementale Regelvorrichtung die Federvorspannung erhöhen oder senken kann, ohne die Federkonstante der Feder wesentlich zu beeinflussen.

27. Ventil nach Anspruch 4, dadurch gekennzeichnet, daß das Hemmungselement stumpfe Zähne hat, die so geformt sind, daß sie mit den Zähnen des Zahnrades zusammenlaufen.

28. Ventil nach Anspruch 4, dadurch gekennzeichnet, daß das Hemmungselement Zähne in Form langer, dünner Elemente (210,211) hat, die dafür ausgelegt sind, durch schuttartige Ablagerungen hindurchzustechen, die sich zwischen den Zähnen des Zahnrads angesammelt haben.

29. Ventil nach einem der Ansprüche 4 bis 16, dadurch gekennzeichnet, daß das (die) Hemmungselement(e) zwei Zähne hat (haben).

30. Chirurgisch implantierbares Nebenschlußventil (314) für die Drainage zerebrospinaler Flüssigkeit in der Behandlung von Hydrocephalus oder zur Ableitung anderer Körperflüssigkeiten, umfassend: ein Gehäuse (322) aus chirurgisch implantierbarem Material; eine Eingangs- (360) und eine Ausgangskammer (362) innerhalb dieses Gehäuses, wobei das Gehäuse Eingangs- und Ausgangsöffnungen umfaßt, die mit der Eingangs- bzw. Ausgangskammer kommunizieren, um die Eingangs- bzw. Ausgangskammer mit externen Kathetern oder anderen Flüssigkeitskanälen zu verbinden; eine Öffnung (326) zwischen den Kammern, wobei diese Öffnung kreisförmig ist und einen kreisförmigen Ventilsitz bildet; ein Ventilglied (332) mit einem Durchmesser, der größer ist als der kreisförmige Ventilsitz, derart, daß es in den Ventilsitz hineinpaßt und ihn verschließt; und ein Federelement (334), um das Ventilglied mit Vorspannung gegen den kreisförmigen Ventilsitz zu pressen, wobei das Federelement dafür ausgelegt ist, die Öffnung geschlossen zu halten, bis der Flüssigkeitsdruck in der Eingangskammer einen vorher festgelegten Ventilöffnungsdruck übersteigt, und gleichzeitig dafür ausgelegt ist, das Ventil zu öffnen, wenn der Ventilöffnungsdruck überschritten wird, und so Flüssigkeit durch die Öffnung in die Ausgangskammer abzuleiten;
**dadurch gekennzeichnet,**
daß das Ventil weiterhin eine magnetische inkrementale Regelvorrichtung umfaßt, die dafür ausgelegt ist, den Betrag der Vorspannung in endlichen Schritten zu erhöhen oder zu senken entsprechend den Impulsen eines applizierten magnetischen Feldes, derart, daß dadurch der Ventilöffnungsdruck in endlichen Schritten erhöht oder gesenkt wird, wobei die magnetische inkrementale Regelvorrichtung ein Element (372), das dafür ausgelegt ist, entsprechend einem externen magnetischen Feld zu rotieren, und eine Nocke (366) von der Gestalt einer kreisförmigen Treppe aufweist, wobei die Federelemente einen Arm (364) aufweisen, der auf dem treppenförmigen Teil der Nocke aufliegt.

31. Vorrichtung für die Drainage zerebrospinaler Flüssigkeit bei der Behandlung von Hydrocephalus oder zur Ableitung anderer Körperflüssigkeiten, umfassend: ein chirurgisch implantierbares Nebenschlußventil nach einem der vorhergehenden Ansprüche; eine Regelvorrichtung (80; 390), die dafür ausgelegt ist, ein getaktetes magnetisches Feld zur Einstellung des Nebenschlußventils bereitzustellen, wobei die Regelvorrichtung einen Elektromagneten (82,84; 392,393, 394,395) sowie Vorrichtungen umfaßt, die geeignet sind, die Regelvorrichtung und dadurch den Magneten entsprechend dem Ventil in einer vorher festgelegten Weise derart auszurichten, daß die Erregung des Elektromagneten eine vorhersagbare Wirkung auf den Ventilöffnungsdruck des Ventils hat.

32. Vorrichtung nach Anspruch 31, dadurch gekennzeichnet, daß die Ausrichtvorrichtung eine Ausnehmung umfaßt, die derart geformt ist, daß sie auf den Vorsprung paßt, der von dem implantierten Ventil auf der Haut eines Patienten gebildet wird.

33. Vorrichtung nach Anspruch 32, dadurch gekennzeichnet, daß die Ausrichtvorrichtung eine Markierung zur Anzeige der Flußrichtung der Flüssigkeit umfaßt, derart, daß der Benutzer die Regelvorrichtung so ausrichten kann, daß der Markierungspfeil in die Flußrichtung der Flüssigkeit durch das Ventil zeigt.

34. Vorrichtung nach einem der Ansprüche 31, 32 oder 33, dadurch gekennzeichnet, daß die Regelvorrichtung zwei Elektromagneten umfaßt, von denen jeder einzeln unter Strom gesetzt werden und in einem Winkel zum anderen ausgerichtet werden kann, derart, daß eine Erhöhung des Ventildruckes durch die Erregung des einen der beiden Elektromagneten und eine Senkung des Ventildruckes durch die Erregung des anderen Elektromagneten erreicht werden kann.

## Revendications

1. Soupape de dérivation (14 ; 100 ; 314) implantable chirurgicalement, destinée à l'évacuation du fluide cérébrospinal dans le traitement de l'hydrocéphalie ou à la dérivation d'autres fluides corporels, comprenant un boîtier (22 ; 102 ; 104 ; 322) formé d'un matériau implantable chirurgicalement, une chambre d'entrée (360) et un chambre de sortie (362) dans le boîtier, le boîtier ayant des orifices d'entrée et de sortie communiquant avec les chambres d'entrée et de sortie respectivement et destinés à raccorder les chambres d'entrée et de sortie à des cathéters extérieurs ou à d'autres conduits de fluide, une ouverture (26 ; 108 ; 326, 328) communiquant avec les chambres, l'ouverture ayant une périphérie circulaire formant un siège circulaire d'obturateur, un organe obturateur (28 ; 110 ; 332) dont le diamètre est supérieur à celui du siège circulaire d'obturateur afin qu'il puisse s'ajuster dans le siège circulaire d'obturateur et le ferme de manière étanche, et un dispositif à ressort (30 ; 116 ; 334) destiné à rappeler l'organe obturateur contre le siège circulaire d'obturateur, le dispositif à ressort étant destiné à maintenir l'ouverture fermée jusqu'à ce que la pression du fluide dans la chambre d'entrée dépasse une pression prédéterminée de soulèvement et à ouvrir l'ouverture lorsque la pression de soulèvement est dépassée afin que le fluide soit évacué par l'ouverture vers la chambre de sortie, la soupape étant caractérisée en ce qu'elle comporte en outre un dispositif d'ajustement magnétique progressif destiné à augmenter ou réduire l'amplitude de la force de rappel par quantités finies en fonction d'impulsions de champ magnétique appliquées depuis l'extérieur d'un corps dans lequel est implantée la soupape, afin que la pression de soulèvement soit augmentée ou réduite de quantités élémentaires finies, le dispositif d'ajustement magnétique progressif comprenant un organe primaire (46 ; 118 ; 132 ; 366, 372) destiné à coopérer avec le dispositif à ressort afin qu'il augmente ou réduise la force de rappel lorsque l'organe primaire est déplacé, l'organe primaire n'étant pas lui-même susceptible de se déplacer sous l'action du champ magnétique externe, et un organe secondaire (48 ; 136, 138, 140, 142 ; 212 ; 378, 380) sensible au champ magnétique appliqué et monté et positionné par rapport à l'organe primaire de manière qu'il puisse se déplacer par rapport à l'organe primaire et provoque au maximum un déplacement élémentaire de l'organe primaire à chaque impulsion du champ magnétique.

2. Soupape selon la revendication 1, caractérisée en outre en ce qu'un dispositif d'arrêt (370) est destiné à arrêter le déplacement de l'organe primaire en position correspondant à une pression connue de soulèvement de manière qu'il permette l'exécution d'ajustement de la pression d'abord par déplacement de l'organe primaire contre le dispositif d'arrêt puis par déplacement élémentaire de l'organe primaire par rapport à l'organe d'arrêt.

3. Soupape selon la revendication 1 ou 2, caractérisée en outre en ce que l'organe primaire comprend un dispositif à vis ou came (118 ; 200 ; 366) qui peut tourner afin que la force de rappel augmente ou diminue.

4. Soupape selon la revendication 3, caractérisée en outre en ce que l'organe primaire comprend un pignon denté (46 ; 132) monté afin qu'il puisse tourner et couplé au dispositif à vis ou à came, et l'organe secondaire comporte un élément à échappement (62 ; 136, 138 ; 210, 211) coopérant avec les dents du pignon et un aimant permanent (54 ; 140, 142 ; 212) destiné à déplacer l'élément à échappement sous l'action du champ magnétique pulsé appliqué afin que le pignon denté tourne.

5. Soupape selon la revendication 4, caractérisée en outre en ce que l'élément à échappement (62) est monté sur un bras (52), en ce que le bras pivote autour d'un axe, et en ce qu'un matériau ferromagnétique ou d'aimant permanent (54) est fixé au bras à un emplacement radial plus éloigné du pivot que l'élément à échappement.

6. Soupape selon la revendication 5, caractérisée en outre en ce que le dispositif à ressort (56) est destiné à ramener l'élément à échappement et le bras en position neutre.

7. Soupape selon la revendication 6, caractérisée en outre en ce que le bras (52), l'élément à échappement (62), le pignon (46) et le dispositif à ressort (56) sont réalisés afin que l'application d'un champ magnétique variable qui augmente puis diminue ait pour effet de déplacer le bras au cours d'un cycle comprenant un déplacement dans un premier sens depuis la position neutre vers une position d'arrêt puis en arrière à nouveau et de manière que, à la suite du déplacement du pignon qui tourne d'un angle fini, la partie de ressort soit soulevée ou abaissée d'une distance finie, et la pression de soulèvement varie d'un mouvement fini.

8. Soupape selon la revendication 7, caractérisée en outre en ce que plus de dix cycles sont nécessaires pour l'élévation de la pression de soulèvement de sa valeur minimale à sa valeur maximale.

9. Soupape selon la revendication 4, caractérisée en outre en ce qu'elle comprend deux éléments à échappement (136, 138 ; 210, 211) coopérant avec le pignon (132) à différentes positions angulaires autour du pignon.

10. Soupape selon la revendication 9, caractérisée en ce que les emplacements angulaires des axes de rotation des éléments à échappement sont séparés angulairement autour du pignon par un nombre entier de dents de pignon augmenté d'une demi-dent.

11. Soupape selon la revendication 9 ou 10, caractérisée en outre en ce que les deux éléments à échappement sont séparés de 180° environ autour du pignon.

12. Soupape selon la revendication 9 ou 10, caractérisée en ce que les deux éléments à échappement sont positionnés afin qu'ils soient séparés de 90° environ autour du pignon.

13. Soupape selon l'une quelconque des revendications 9 à 12, caractérisée en outre en ce que les deux éléments à échappement (136, 138) sont connectés chacun à un aimant permanent (140, 142), et chaque paire comprenant un aimant et un élément à échappement pivote autour d'un axe (144) passant entre les deux pôles de l'aimant.

14. Soupape selon les deux revendications 11 et 13, caractérisée en outre en ce que, pour chaque aimant, la droite reliant les pôles de l'aimant est parallèle de façon générale à la droite de l'autre des aimants.

15. Soupape selon les deux revendications 12 et 13, caractérisée en outre en ce que, pour chaque aimant, la droite reliant les pôles de l'aimant est perpendiculaire de façon générale à la droite de l'autre des aimants.

16. Soupape selon l'une quelconque des revendications 9 à 15, caractérisée en outre en ce que les éléments à échappement sont positionnés de manière qu'un champ magnétique externe puisse être appliqué à un premier emplacement tel que le champ donne un moment de rotation d'un élément à échappement plus intense que celui de l'autre, et de manière que, lorsque le champ extérieur est appliqué à un second emplacement, un moment de rotation plus intense soit appliqué à l'autre des éléments à échappement.

17. Soupape selon l'une quelconque des revendications précédentes, caractérisée en outre en ce que le mouvement de l'organe primaire est transformé en un mouvement d'une partie du dispositif à ressort, et en ce que le dispositif à ressort comprend un ressort à trois bras ayant deux bras latéraux (36, 38 ; 122 ; 340, 342) et un bras central (32 ; 120 ; 338), une première extrémité du bras central (34 ; 336) étant disposée afin qu'elle rappelle l'organe obturateur contre le siège circulaire d'obturateur, et l'autre extrémité du bras central est ladite partie du dispositif à ressort.

18. Soupape selon la revendication 17, caractérisée en outre en ce que les extrémités des bras latéraux (122 ; 340, 342) sont retenues par un étrier (126 ; 346) disposé au-dessus des surfaces supérieures des extrémités pour la retenue de celles-ci afin qu'elles ne puissent pas se déplacer vers le haut.

19. Soupape selon la revendication 18, caractérisée en outre en ce que l'étrier (126 ; 346) dépasse sous les extrémités des bras latéraux (122 ; 340, 342), le long des bords internes (123) des bras, et sur lesdites extrémités, si bien que les extrémités sont retenues contre tout déplacement latéral aussi bien qu'ascendant.

20. Soupape selon la revendication 19, caractérisé en outre en ce que l'étrier a une forme en U afin qu'il passe entre le bras central et les bras latéraux.

21. Soupape selon les revendications 19 ou 20, caractérisée en outre en ce que chaque extrémité des bras latéraux (122) a une encoche (123) logeant la partie d'étrier dans laquelle passent les bords internes des bras, si bien que le ressort est retenu contre tout déplacement longitudinal ainsi que contre un déplacement vers le haut et vers le côté.

22. Soupape selon l'une quelconque des revendications 18 à 21, caractérisée en outre en ce que l'étrier n'est pas fixé aux extrémités des bras latéraux mais est disposé sur elles avec du jeu.

23. Soupape selon l'une quelconque des revendications 17 à 22, lorsqu'elles dépendent de la revendication 3, caractérisée en outre en ce que le dispositif à came ou vis comporte une vis passant à travers la partie de ressort (figure 2).

24. Soupape selon l'une quelconque des revendications 17 à 22, caractérisée en outre en ce que le dispositif à came ou vis comporte une surface de came (200) destinée à soulever la partie de ressort (120) lorsque le pignon (132) tourne.

25. Soupape selon l'une quelconque des revendications 1 à 16, caractérisée en outre en ce que l'organe primaire est le rotor (372) d'un moteur pas à pas, et l'organe secondaire comprend plusieurs éléments (378) de stator formés d'un matériau perméable et faiblement ferromagnétique, dont la configuration et la position par rapport au rotor sont telles que, lorsqu'ils sont aimantés sous l'action du champ magnétique extérieur, les éléments de stator assurent le renforcement et l'orientation du champ magnétique à leur voisinage et provoquent un déplacement du rotor, et en ce que le déplacement de l'organe primaire est transformé en un déplacement d'une partie du dispositif à ressort (364) par une came (366) ayant la forme d'un escalier circulaire, une partie du dispositif à ressort étant au contact de l'escalier, et la rotation du rotor provoque une rotation de la came et un changement de position de la partie à ressort le long de l'escalier.

26. Soupape selon l'une quelconque des revendications précédentes, caractérisée en outre en ce que le dispositif à ressort comporte un ressort ayant une faible raideur de manière que la pression de soulèvement soit pratiquement indépendante des variations du débit de fluide, et en ce que le dispositif d'ajustement magnétique progressif est destiné à augmenter ou réduire la force de rappel sans affecter notablement la raideur du ressort.

27. Soupape selon la revendication 4, caractérisée en outre en ce que l'élément à échappement a des dents émoussées dont la configuration correspond à celle des dents du pignon.

28. Soupape selon la revendication 4, caractérisée en outre en ce que l'élément à échappement a la forme de longs éléments minces (210, 211) destinés à percer les débris collectés entre les dents du pignon.

29. Soupape selon l'une quelconque des revendications 4 à 16, caractérisée en outre en ce que l'élément ou les éléments à échappement ont deux dents.

30. Soupape (314) de dérivation implantable chirurgicalement destinée à l'évacuation du fluide cérébrospinal dans le traitement de l'hydrocéphalie ou pour la dérivation d'autres fluides corporels, comprenant un boîtier (322) construit en un matériau implantable chirurgicalement, une chambre d'entrée (360) et une chambre de sortie (362) placées dans le boîtier, le boîtier ayant des orifices d'entrée et de sortie communiquant avec les chambres d'entrée et de sortie respectivement et destinés à raccorder les chambres d'entrée et de sortie à des cathéters extérieurs ou d'autres conduits de fluide, une ouverture (326) faisant communiquer les chambres, l'ouverture ayant une périphérie circulaire formant un siège circulaire d'obturateur (328), un organe obturateur (332) dont le diamètre est supérieur à celui du siège circulaire d'obturateur de manière qu'il puisse s'ajuster dans le siège circulaire d'obturateur et le ferme de manière étanche, et un dispositif à ressort (334) destiné à rappeler l'organe obturateur contre le siège circulaire d'obturateur, le dispositif à ressort étant destiné à maintenir l'ouverture fermée jusqu'à ce que la pression du fluide dans la chambre d'entrée dépasse une pression prédéterminée de soulèvement et à ouvrir l'ouverture lorsque la pression de soulèvement est dépassée afin que le fluide soit évacué par l'ouverture vers la chambre de sortie, la soupape étant caractérisée en ce qu'elle comporte en outre un dispositif d'ajustement magnétique progressif destiné à augmenter ou réduire l'amplitude de la force de rappel par quantités élémentaires finies en fonction d'impulsions d'un champ électrique appliquées de manière que la pression de soulèvement soit augmentée ou réduite par quantités élémentaires finies, le dispositif d'ajustement magnétique progressif comprenant un élément (372) destiné à tourner sous l'action d'un champ magnétique externe, et une came (366) sous forme d'un escalier circulaire, le dispositif à ressort comprenant un bras (364) supporté sur l'escalier.

31. Appareil d'évacuation du fluide cérébrospinal dans le traitement de l'hydrocéphalie ou pour la mise en dérivation d'autres fluides corporels, l'appareil étant caractérisé en ce qu'il comprend une soupape de dérivation implantable chirurgicalement selon l'une quelconque des revendications précédentes, et un appareil (80, 390) d'ajustement destiné à appliquer un champ magnétique pulsé d'ajustement de la soupape de dérivation, l'appareil d'ajustement comprenant un électro-aimant (82, 84 ; 392, 393, 394, 395) et un dispositif destiné à orienter l'appareil et ainsi l'aimant à une orientation prédéterminée par rapport à la soupape afin que l'excitation de l'électro-aimant ait un effet prévisible sur la pression de soulèvement de la soupape.

32. Appareil selon la revendication 31, caractérisé en outre en ce que le dispositif d'orientation comporte une cavité ayant une configuration telle qu'elle s'ajuste sur la saillie qui peut être formée à la surface de la peau d'un patient par la soupape lorsque celle-ci est implantée au-dessous.

33. Appareil selon la revendication 32, caractérisé en outre en ce que le dispositif d'orientation comporte un marquage destiné à indiquer la direction de circulation du fluide afin que l'utilisateur puisse orienter l'appareil d'ajustement de manière que la flèche soit tournée dans le sens d'écoulement du fluide dans la soupape.

34. Appareil selon l'une des revendications 31, 32 et 33, caractérisé en outre en ce que l'appareil d'ajustement comporte deux électro-aimants qui peuvent être excités chacun séparément et qui sont orientés chacun suivant un angle par rapport à l'autre afin que l'ajustement à une valeur supérieure de la pression de soulèvement puisse être réalisé par excitation de l'un des électro-aimants et que l'ajustement à une valeur inférieure puisse être réalisé par excitation de l'autre des électro-aimants.
